# EUROPÄISCHE PATENTANMELDUNG

(11) **EP 4 053 112 A1**
(43) Veröffentlichungstag der Anmeldung: **07.09.2022**
(21) Anmeldenummer: 21160525.8
(22) Anmeldetag: 03.03.2021
(51) Int. Cl.: C07D 307/32

(54) **VERFAHREN ZUR HERSTELLUNG VON SUBSTITUIERTEM 4-OXO-TETRAHYDROFURAN**

(71) Anmelder: Bayer AG, 51373 Leverkusen (DE)
(72) Erfinder: Die Erfindernennung liegt noch nicht vor
(74) Vertreter: BIP Patents

(57) **Zusammenfassung**

Die vorliegende Erfindung betrifft ein neues Verfahren zur Herstellung von Alkyl-4-oxotetrahydrofuran-2-carboxylat **(I).**

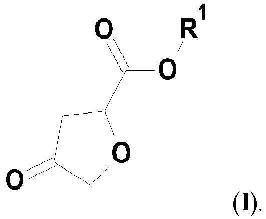

## Beschreibung

Die vorliegende Erfindung betrifft ein neues Verfahren zur Herstellung von Alkyl-4-oxotetrahydrofuran-2-carboxylat (**I**).

Methyl-4-oxotetrahydrofuran-2-carboxylat der Formel (**I**) ist eine wichtige Vorstufe von agrochemischen (vgl. WO2018/228985) Wirkstoffen.

Die Synthese von Methyl-4-oxotetrahydrofuran-2-carboxylat der Formel (**I**) ist bekannt, z.B. aus Helv. Chim. Acta 1959, 1177 und WO 2016/205633. Jedoch sind ausgehend von Dimethyl(*Z*)-but-endioate drei Reaktionsschritte notwendig, um Methyl-4-oxotetrahydrofuran-2-carboxylat der Formel (**I**) herzustellen, was mit einem Ausbeuteverlust einhergeht. Des Weiteren sind die Reagenzien, die im Stand der Technik verwendet werden (z.B. Natriumpulver, NaH, TMSCHN₂, CH₂N₂) nicht für eine großtechnische Synthese geeignet, da der sichere Umgang mit diesen Chemikalien im großen Maßstab schwierig ist oder sie sehr toxisch sind.

Im Lichte des zuvor beschriebenen Standes der Technik liegt der vorliegenden Erfindung die Aufgabe zugrunde, ein Verfahren zur Herstellung der Verbindungen der allgemeinen Formel (**I**) ausgehend von Verbindungen der allgemeinen Formel (**II**) und (**III**) in nur zwei Reaktionsschritten zu entwickeln, das auch für die Produktion in großem Maßstab geeignet ist.

Die zuvor beschriebene Aufgabe wird gelöst durch ein Verfahren zur Herstellung von Verbindungen der allgemeinen Formel (**I**) 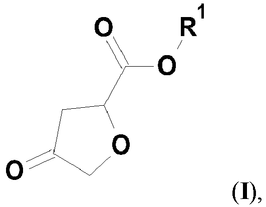 worin
R¹ (C₁-C₄)-Alkyl ist,
dadurch gekennzeichnet, dass Verbindungen der allgemeinen Formel (**II**) 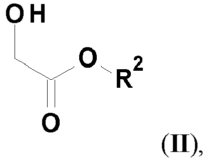 worin
R² (C₁-C₄)-Alkyl ist,
mit Verbindungen der allgemeinen Formel (**III**) 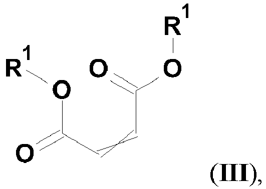 worin R¹ wie oben definiert ist,
durch Zugabe von **M**O*^{t}*Bu,
worin
M ein Alkalimetallion ist,
Zyklisierungsprodukte mit der allgemeinen Formel (**IV**) ergeben 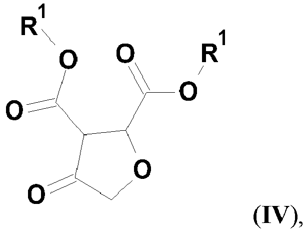 worin R¹ wie oben definiert ist,
welche unter nicht hydrolytischen Bedingungen eine Dealkoxycarbonylierung unterlaufen und zu Verbindungen der allgemeinen Formel (**I**) reagieren.

Bevorzugte Restedefinitionen für die Verbindungen der allgemeinen Formeln (**I**), (**II**), (**III**), (**IV**) und MO*^{t}*Bu sind die folgenden:
- R¹: steht für Ethyl oder Methyl,
- R²: steht für Ethyl oder Methyl,
- M: steht für Natrium oder Kalium.

Besonders bevorzugte Restedefinitionen für die Verbindungen der allgemeinen Formeln (**I**), (**II**), (**III**), (**IV**) und **M**O*^{t}*Bu sind die folgenden:
- R¹: steht für Methyl,
- R²: steht für Methyl,
- M: steht für Natrium.

### Erläuterung des Verfahrens

Die Reaktionsbedingungen zur Herstellung von Verbindungen der allgemeinen Formel (**I**) werden im Folgenden im Detail erläutert.

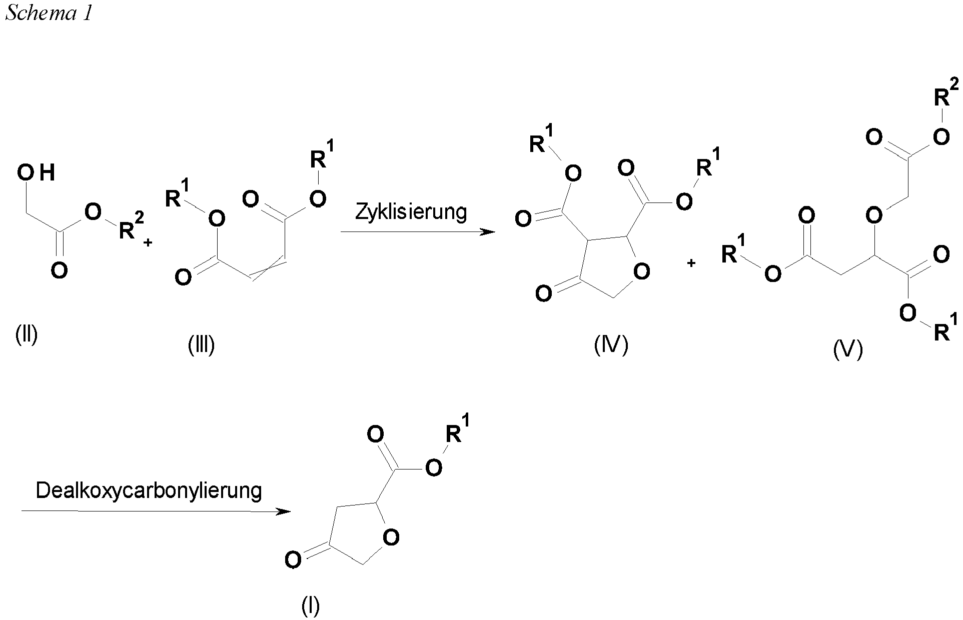

Die Verbindungen der allgemeinen Formel (**II**) reagieren mit Verbindungen der allgemeinen Formel (**III**) in Anwesenheit von **M**O*^{t}*Bu zu Zyklisierungsprodukten mit der allgemeinen Formel (**IV**), welche unter nicht hydrolytischen Bedingungen eine Dealkoxycarbonylierung unterlaufen und zu Verbindungen der allgemeinen Formel (**I**) reagieren.

Nach der Zyklisierung kann neben dem eigentlichen Produkt, den Verbindungen der allgemeinen Formel (**IV**), auch noch Edukt der allgemeinen Formel (**III**) und das Intermediat der allgemeinen Formel (**V**) im Reaktionsgemisch enthalten sein.

Die Verbindungen der allgemeinen Formel (**II**) und (**III**) sind kommerziell erhältlich. Die Verbindungen der allgemeinen Formel (**III**) können als *E*-oder *Z*-Isomer eingesetzt werden. Unter den Reaktionsbedingungen findet eine Isomerisierung zwischen *Z*- und *E*-Isomeren statt. Bevorzugt wird das Z-Isomer umgesetzt.

Die Verbindungen der allgemeinen Formel (**I**) weisen ein Stereozentrum auf. Folglich liegt das Produkt in Form eines Racemates vor.

### Zyklisierung:

Die Zyklisierung ist aus dem Stand der Technik bekannt und wird dort mit NaH oder Natriumpulver durchgeführt (Helv. Chim. Acta 1959, 1177; WO 2016/205633). Diese Reagenzien sind nicht für eine großtechnische Synthese geeignet, da der sichere Umgang mit ihnen im großen Maßstab schwierig ist.

Die Ausbeute des erfindungsgemäßen Verfahrens ist höher (> 30%) als die des im Stand der Technik beschriebenen Verfahrens mit NaH bzw. Natriumpulver (< 30%). Außerdem ist die Reaktion wegen der *tert*-Butoxid Base auch großtechnisch einsetzbar.

Vorteilhaft für das Erzielen einer hohen Ausbeute ist das langsame Zugeben von **M**O*^{t}*Bu.

Bevorzugt erfolgt das Zugeben von **M**OtBu über 0,5 bis 8 Stunden, besonders bevorzugt über 3 bis 5 Stunden.

Das molare Verhältnis von **M**OtBu bezogen auf Verbindungen der allgemeinen Formel (**II**) beträgt 0,8 bis 3 Äquivalente, bevorzugt 0,9 bis 1,2 Äquivalente.

Das molare Verhältnis von Verbindungen der allgemeinen (**II**) zu Verbindungen der allgemeinen Formel (**III**) beträgt 0,8 bis 3 Äquivalente, bevorzugt 0,9 bis 1,2 Äquivalente.

Die Temperatur kann in einem breiten Bereich variiert werden und ist z.B. abhängig vom Lösungsmittel. Sie liegt für die Reaktion bevorzugt bei 0°C bis 70°C, ganz besonders bevorzugt bei 40°C bis 60°C.

Die Reaktion wird gewöhnlich in einem Lösemittel durchgeführt, bevorzugt in THF, Toluol oder Me-THF. Bevorzugt handelt es sich um wasserfreies ("trockenes" oder absolutes) Lösungsmittel.

Dealkoxycarbonylierung (Organic Reactions, Vol 81):
Während mit Schwefelsäure in Wasser (s. Helv. Chim. Acta 1959, 1177; WO 2016/205633) die Esterspaltung/Decarboxylierung zu 4-Oxotetrahydrofurancarbonsäure stattfindet, kann unter nicht hydrolytischen, z.B. wasserfreien Reaktionsbedingungen, der Ester in Verbindungen der allgemeinen Formel (**I**) bestehen bleiben, sodass der zusätzliche Schritt der erneuten Veresterung, der im Stand der Technik mit CH₂N₂ vorgenommen wird, entfallen kann. Dadurch entfallen auch die im Stand der Technik notwendigen zahlreichen Extraktionen der 4-Oxotetrahydrofurancarbonsäure, die schwer aus Wasser zu isolieren ist.

Die Ausbeute kann in der Folge wesentlich gesteigert werden (>95% gegenüber 75% mit Schwefelsäure im Stand der Technik). Auf toxische Reagenzien, wie z.B. Diazomethan, kann verzichtet werden.

Das Reagenz (s. Tabelle 1) wird im Überschuss eingesetzt, gegebenenfalls in Kombination mit einem Lösungsmittel. Bevorzugt wird das Reagenz auch als Lösungsmittel verwendet.

Die Temperatur für die Reaktion ist abhängig vom Reagenz bzw. Lösungsmittel.

In Tabelle 1 sind beispielhaft einige dieser Reaktionsbedingungen aufgeführt, ohne sie jedoch auf diese zu beschränken.

**Tabelle 1: Dealkoxycarbonylierung**

| **Bedingungen (Reagenz = Lösungsmittel)** | **Temp/°C** | **Zeit/Stunden** |
|---|---|---|
| Essigsäure | 118 | 6 |
| Propionsäure | 140 | 18 |
| B(OH)₃ | 145-175 | 4 |

### Beispiele

Die vorliegende Erfindung wird anhand der nachfolgenden Beispiele näher erläutert, ohne die Erfindung dabei auf diese einzuschränken.

### Messverfahren

Die Produkte wurden mittels ¹H-NMR charakterisiert.

### Beispiel 1

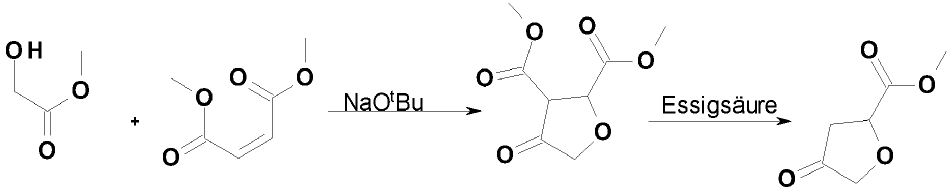

### Methyl-4-oxotetrahydrofuran-2,3-dicarboxylat

108 g Methylglycolat (1.2 mol) und 172 g Dimethylmaleat (1.2 mol) werden zusammen mit 800 ml THF in einen Kessel mit Heiz/Kühlmantel (2 1) gegeben. Die Mischung wird auf 50°C erwärmt und dann wird eine Lösung von 120 g NaO*^{t}*Bu (1.25 mol) in 800 mL THF über 3 Stunden hinzugefügt. Während der ersten Minuten steigt die Innentemperatur auf 53°C und bleibt dann bei 50°C. Nach Zugabe von ca. 15% der Base wird das Reaktionsgemisch trübe und ein Feststoff fällt aus. Nach dem Ende der Zugabe der Base wird das Reaktionsgemisch noch für 1 Stunde bei 50°C gerührt und wird dann auf -1°C abgekühlt. Dann werden 215 g Essigsäure (3.6 mol) hinzugefügt, und zwar über 20 Minuten. Die Temperatur der Reaktionsmischung steigt auf 6°C. Dann wird das Reaktionsgemisch auf 1°C abgekühlt und 47.1 g HCl-Gas werden unter die Oberfläche eingeführt, und zwar über 45 Minuten. Die Temperatur der Reaktionsmischung steigt auf 8.5°C. Das Lösungsmittel THF wird bei einem Druck von 50 mbar und einer Temperatur von 50°C destillativ entfernt. Zurück bleibt ein Öl.

### Methyl-4-oxotetrahydrofuran-2-carboxylat

Das Öl aus dem vorhergehenden Reaktionsschritt wird in 200 ml Essigsäure gegeben und der entstehende Feststoff wird abfiltriert. Das Filtrat wird auf 118°C erhitzt, und zwar für 6 Stunden. Dann wird die Essigsäure bei 50°C und einem Druck von 4 mbar abdestilliert, Das Produkt wird destillativ gereinigt. Das Produkt ist ein Öl (87.4 g, 46% Ausbeute).
¹H-NMR (600MHz, DMSO-d6): δ (ppm) = 4.97 (dd, *J* = 8.8, 5.1 Hz, 1H), 4.02 (d, *J* = 16.7 Hz, 1H), 3.99 (d, *J* = 16.7 Hz, 1H), 3.69 (s, 3H), 2.88 (dd, *J* = 18.2, 8.8 Hz, 1H), 2.60 (dd, *J* = 18.2, 5.1 Hz, 1H).

**Tabelle 2: Vergleich der Ausbeute**

| | **Reaktionsbedingungen** | **Gesamtausbeute** |
|---|---|---|
| **Beispiel Nr.1** | NaO*^{t}*Bu/Essigsäure | **46%** |
| **Vergleichsbeispiel:** Helv. Chim. Acta **1959**, 1177 | Natriumpulver, Schwefelsäure, Wasser, CH₂N₂ | **23%** |

## Patentansprüche

1. Verfahren zur Herstellung von Verbindungen der allgemeinen Formel (**I**) 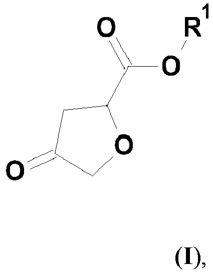 worin
R¹ (C₁-C₄)-Alkyl ist,
**dadurch gekennzeichnet, dass** Verbindungen der allgemeinen Formel (**II**) 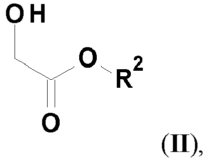 worin
R² (C₁-C₄)-Alkyl ist,
mit Verbindungen der allgemeinen Formel (**III**) 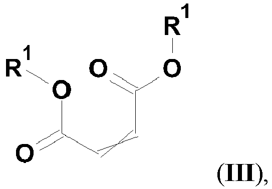 worin R¹ wie oben definiert ist,
durch Zugabe von **M**O*^{t}*Bu,
worin
M ein Alkalimetallion ist,
Zyklisierungsprodukte mit der allgemeinen Formel (**IV**) ergeben 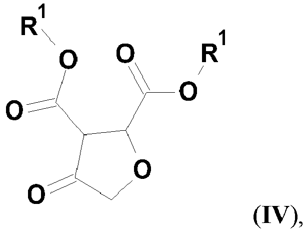 worin R¹ wie oben definiert ist,
welche unter nicht hydrolytischen Bedingungen eine Dealkoxycarbonylierung unterlaufen und zu Verbindungen der allgemeinen Formel (**I**) reagieren.

2. Verfahren nach Anspruch 1, **dadurch gekennzeichnet, dass** die Restedefinitionen für die Verbindungen der allgemeinen Formeln (**I**), (**II**), (**III**), (**IV**) und **M**O*^{t}*Bu die folgenden sind:
R¹ steht für Ethyl oder Methyl,
R² steht für Ethyl oder Methyl,
M steht für Natrium oder Kalium.

3. Verfahren nach Anspruch 1, **dadurch gekennzeichnet, dass** die Restedefinitionen für die Verbindungen der allgemeinen Formeln (**I**), (**II**), (**III**), (**IV**) und **M**O*^{t}*Bu die folgenden sind:
R¹ steht für Methyl,
R² steht für Methyl,
M steht für Natrium.

4. Verfahren nach einem der Ansprüche 1 bis 3, **dadurch gekennzeichnet, dass** die Zyklisierung bei 0°C bis 70°C durchgeführt wird.

5. Verfahren nach einem der Ansprüche 1 bis 3, **dadurch gekennzeichnet, dass** die Zyklisierung bei 40°C bis 60°C durchgeführt wird.

6. Verfahren nach einem der Ansprüche 1 bis 5, **dadurch gekennzeichnet, dass** das Lösungsmittel von der Zyklisierung THF, Toluol oder Me-THF ist.

7. Verfahren nach Anspruch 6 **dadurch gekennzeichnet, dass** für die Zyklisierung wasserfreies Lösungsmittel verwendet wird.

8. Verfahren nach einem der Ansprüche 1 bis 7, **dadurch gekennzeichnet, dass** das Z-Isomer der Verbindung der allgemeinen Formel (**III**) eingesetzt wird.

9. Verfahren nach einem der Ansprüche 1 bis 8, **dadurch gekennzeichnet, dass M**O*^{t}*Bu bei der Zyklisierung über 0,5 bis 8 Stunden zugegeben wird.

10. Verfahren nach einem der Ansprüche 1 bis 9, **dadurch gekennzeichnet, dass M**O*^{t}*Bu bei der Zyklisierung zudosiert wird.

11. Verfahren nach einem der Ansprüche 1 bis 10, **dadurch gekennzeichnet, dass** das Reagenz bzw. Lösungsmittel der Dealkoxycarbonylierung AcOH ist.
